# EUROPÄISCHE PATENTSCHRIFT

(11) **EP 0 698 005 B1**
(45) Veröffentlichungstag und Bekanntmachung des Hinweises auf die Patenterteilung: **02.04.1997**
(21) Anmeldenummer: 94913008.2
(22) Anmeldetag: 19.04.1994
(51) Int. Cl.: C05F 3/00, C05F 7/00, C05F 17/00

(54) **VERFAHREN UND VORRICHTUNG ZUR KOMPOSTIERUNG UND NASSVERGÄRUNG VON BIOLOGISCHEN ABFÄLLEN**
DEVICE AND METHOD FOR THE COMPOSTING AND WET-FERMENTATION OF BIOLOGICAL WASTE
PROCEDE ET DISPOSITIF POUR LE COMPOSTAGE ET LA FERMENTATION PAR VOIE HUMIDE DE DECHETS BIOLOGIQUES

(30) Priorität: 22.04.1993 CH 1267/93; 06.08.1993 CH 2360/93; 19.03.1994 DE 4409539
(43) Veröffentlichungstag der Anmeldung: 28.02.1996
(73) Patentinhaber: BEG BIOENERGIE GESELLSCHAFT MBH, 45127 Essen (DE)
(72) Erfinder: KANITZ, Jürgen, D-44805 Bochum (DE); KESSELRING, Bruno, CH-7205 Zizers (CH)
(74) Vertreter: Vomberg, Friedhelm, Dipl.-Phys.
(86) Internationale Anmeldenummer: DE9400440
(87) Internationale Veröffentlichungsnummer: WO9424071

(56) Entgegenhaltungen:
- EP-A- 0 172 292
- WO-A-92/15540
- DE-A- 4 001 024
- DATABASE WPI Section Ch, Week 8839, Derwent Publications Ltd., London, GB; Class C04, AN 88-276279 & NL,A,8 700 306 (VAN TILBURG A A M) 1. September 1988

## Beschreibung

Die Erfindung betrifft ein Verfahren zur Behandlung von organischen Bio-Reststoffen, insbesondere aus kommunalen und/oder gewerblichen Abfallstoffen einschließlich roher und/oder gekochter Speisereste, landwirtschaftliche Abfallstoffe, insbesondere Tierexkremente und/oder pflanzlicher Bestandteile, bei dem die Bio-Reststoffe in einen Feststoff- und einen Flüssig-Anteil getrennt werden und anschließend der Feststoffanteil einer aeroben Mietenkompostierung und der Flüssig-Anteil einem Anaerob-Reaktor zugeführt wird, von dem das durch Vergärung entstandene Biogas abgezogen wird.

Die Erfindung betrifft ferner eine Vorrichtung zur Gewinnung von Biogas oder Kompost durch Naßvergärung und Kompostieren von organischen Bio-Reststoffen mit einem als Preßschnecken-FestFlüssig-Separator ausgebildeten Feststoffreaktor, der einen Flüssigkeitsaustrag und einen diesem nachgeschalteten Anaerob-Reaktor zur Vergärung mit einem Biogasabzug und eine Kompostiereinrichtung besitzt

Unter Bio-Reststoffen werden neben den bereits genannten Abfallstoffen alle Stoffe verstanden, die einen der Vergärung zugänglichen Gehalt an Kohlenstoff in gelöster Form (DOC) aufweisen. Dies können beispielsweise pflanzliche Grünabfälle, wie Langgras, Laub, Rasenschnitt, Astwerk, Gehölzschnitt, Blumen, Ernterückstände aus dem Garten/Landschaftsbau, aber auch Abfälle aus der gemüse- und obstverarbeitenden Industrie sein, wie Schalen von Früchten, Ölfrüchten, Trester, Bierschlämme, Hefen, Kokosfasern, Blätter und Strünke aus der Konservenindustrie, Kartoffelschalen und Blattrückstände, Rinden und Späne aus der Holz- und Papierindustrie, organischer kommunaler oder gewerblicher Stadtmüll und schließlich landwirtschaftliche Abfälle.

Seit langem ist es bekannt, reine Grünabfälle zu kompostieren, wozu die zerkleinerten Abfälle mit oder ohne Zugabe von Kompostzuschlägen in Kompostmieten aufgesetzt und einem Verrottungsprozeß überlassen werden, bei dem die Abfälle allmählich zersetzen. Es ist inzwischen auch bekannt, daß die Verrottungszeit durch Umsetzen der aufgeschichteten Mieten sowie eine gezielte Prozeßführung durch Überwachung der Temperatur und/oder Mietenfeuchtigkeit beschleunigt werden kann.

In der EP 0 429 744 A2 wird ein Verfahren beschrieben, bei dem das Abfallmaterial nach Vorbereitung durch Sieben, Trennen und Zerkleinern einem Gärbehälter zugeführt wird und zum Zwecke der Einleitung und Aufrechterhaltung eines aeroben biologischen Zersetzungsprozesses unter ständigem Rühren allmählich gegen die zentrale Austrittsöffnung des Gärbehälters verschoben wird. Zur Verbesserung dieser Fermentiermethode wird eine aerobe biologische Verrottung biologischer Abfälle vorgeschlagen, bei der die Abfälle unter ständigem Rühren innig mit Luft vermischt und dadurch einer bakteriellen aeroben Zersetzung zugeführt werden und das aus dem Verrottungsprozeß hervorgegangene Produkt dem Aufnahmebehälter entnommen und einer Veredelung unterzogen wird. Hierzu soll der Aufnahmebehälter etwa bis zur Hälfte mit festem Abfall und anschließender Zugabe flüssiger Substanzen gefüllt und während einer Verweilzeit von 2 bis 3 Stunden umgerührt werden, bevor dekantierter Klärschlamm sowie Weißkalk zugegeben wird und das Umrühren bis zu einer Verweilzeit von 69 oder 70 Stunden fortgesetzt wird.

Die WO 92/15540 beschreibt ein Verfahren zur getrennten Behandlung und Entsorgung von Gemengen aus festen und flüssigen, organischen Abfallstoffen, wobei das Gemenge durch mechanische Trennung in eine Flüssigphase mit einem niedrigen Feststoffgehalt in feinstverteilter Form und einen Feststoffanteil mit einem Wassergehalt aufgespalten wird, worauf die Flüssigphase unter Bildung von Biogas einem anaeroben, der Feststoffanteil unter Bildung von Kompost, Dünger oder Futtermittel einem aeroben Fermentationsvorgang unterworfen werden. Die im Biogas und/oder in der Flüssigphase enthaltenen Ballaststoffe sollen durch chemische Maßnahmen, wie Ausfällung bzw. Flockung, entfernt und im Kreislauf geführt werden, bevor das Biogas verbrannt bzw. die Flüssigphase in eine Kläranlage abgelassen oder der weitergehenden Reinigung zugeführt wird. Dieses Verfahren soll für Schweinegülle, Rindergülle, Klärschlamm, Molke oder ähnliche Stoffe anwendbar sein. zur mechanischen Separation in eine Flüssigphase und einen Schlamm wird eine stehende SiebZentrifuge, ein horizontal liegender Preßschnecken-Separator mit einem Sieb oder ein Dekanter vorgeschlagen.

Die EP 0 172 292 A1 betrifft ein Verfahren zur Behandlung organischer Flüssigkeitsabfälle, deren Feststoffanteil abgetrennt und einer aeroben Fermentation zugeführt wird, während der Flüssiganteil eine anaerobe Fermentation durchlaufen soll. Es werden carbonhaltige Abfälle, wie Gehölz, Stallmist, Rinde, Fruchtschalen, Sägespäne etc. einerseits und Nahrungsabfallstoffe andererseits sowie schließlich Feststoffe aus einem Trommelsieb, das zur Trennung von Fest-Flüssigmischungen dient, einem Feinzerkleinerer zugeführt, bevor sie als semifeste Schüttung einer Kompostierung zugeleitet werden. Bei diesem Verfahren wird der Teil der Flüssigkeit verwertet, der bereits in der Fest-Flüssigmischung vorgesehen ist.

Es ist Aufgabe der vorliegenden Erfindung, ein Verfahren der eingangs genannten Art und eine Vorrichtung anzugeben, das bzw. die eine verbesserte Behandlung organischer Reststoffe zur Erzeugung verwertbarer Stoffe, wie Kompost und/oder Biogas ermöglicht.

Diese Aufgabe wird durch das im Anspruch 1 beschriebene Verfahren gelöst.

Im Unterschied zu dem eingangs genannten, nach dem Stand der Technik bekannten Verfahren werden aus den Bio-Reststoffen vor der Fest-Flüssigtrennung unter intensivem Mischen und Homogenisieren die in Wasser gelösten und lösbaren C-haltigen (organischen) Bestandteile (DOC) ausgewaschen.

Durch die Trennung des Abfalls in eine Feststofffraktion und eine Flüssigfraktion, die als eine durch Siebung hergestellte Suspension vorliegt, können eine aerobe Behandlung der Feststofffraktion einerseits und eine anaerobe Behandlung der gelösten und suspensierten Fraktion jeweils besser kontrolliert werden, wozu insbesondere geringere Behältervolumina als bei einer gemeinsamen Behandlung beider Fraktionen in einem Behälter erforderlich sind. Vergleichsweise können die getrennte Kompostierung und Naßvergärung insgesamt bei Erzeugung qualitativ hochwertiger Endprodukte schneller ablaufen.

Weiterbildungen des erfindungsgemäßen Verfahrens sind in den Ansprüchen 2 bis 8 beschrieben.

So wird in der Trennstufe, in der nach Aufgabe des Abfalles eine Feuchte von 70 bis 80 Vol.-% vorliegt, diese Feuchte aufrechterhalten, was selbst nach Abführen der Flüssigsuspension durch Zuführung von Wasser bzw. Prozeßwasser möglich ist. Zumindest sollte die Feuchte von 40 bis 60 Vol.-% in der Trennstufe nicht unterschritten werden, da nur bei relativ hohen Feuchten ein Auswaschen der C-haltigen Bestandteile möglich ist. Vorzugsweise wird das Prozeßwasser rezyklisierend in die Trennstufe geleitet, das nach erfolgter Methanisierung (Vergärung) im Anaerob-Reaktor abfällt.

Eine weitere Optimierung der Prozeßsteuerung in der Trennstufe kann durch gezielte Sauerstoffzuführung und Temperatursteuerung, insbesondere Aufrechterhaltung einer konstanten Temperatur, erreicht werden. Die betreffende Regelung kann in Abhängigkeit gemessener Temperaturen, Sauerstoffgehalte, CO₂-Gehalte im Abgas etc. vorgenommen werden.

Nach der Aufgabe von biologischen Reststoffen in die Trennstufe wird zunächst eine exotherm verlaufende Reaktion einsetzen. Die nicht benötigte überschüssige Wärme wird vorzugsweise aus der Trennstufe abgezogen und dem Anaerob-Reaktor zugeleitet.

Nach einer weiteren Ausgestaltung der Erfindung wird erst nach Unterschreiten eines vorgebbaren Gehaltes an gelösten C-haltigen Bestandteilen in der aus der Trennstufe abgeführten Flüssigkeit die in der Trennstufe volumenmäßig zusammengefallene Reststoffmenge auf einen möglichst niedrigen Restfeuchtegehalt abgepreßt, bevor die gesamte Charge an festen Reststoffen aus der Trennstufe abgeführt und anschließend kompostiert wird. Die Kompostierung kann nach üblichen, nach dem Stand der Technik bekannten Verfahren, mit und ohne Zugabe von Zuschlagstoffen durchgeführt werden.

Eine beschleunigte Trennung bzw. Abtrennung der Flüssigphase aus den biologischen Abfallstoffen erreicht man, wenn die Stoffe gepreßt und/oder abgesiebt werden. Die aus der Trennstufe, d.h. dem Feststoffreaktor, ausgeschleusten Flüssigkeiten liegen als Suspensionen vor. Um den Anaerob-Reaktor nicht mit Feststoffanteilen bzw. Schlämmen zu belasten, wird die betreffende Suspension vor Einleitung in den Anaerob-Reaktor sedimentiert. Vorzugsweise wird der beim Sedimentieren als Schlamm anfallende Feststoff als Animpfmaterial zur Reaktionsbeschleunigung, weiterhin vorzugsweise nach Sauerstoffanreicherung in Form von Belebtschlamm nach frischer Füllung des Feststoffreaktors den Bio-Reststoffen zugeführt.

Die aus der Trennstufe abgeführte Flüssigkeit wird vor der Einleitung in den Anaerob-Reaktor einer Ultraschallbehandlung oder Biogasstrippung zur Entfernung noch enthaltenen Sauerstoffes unterzogen. Durch den völligen bzw. weitgehenden Sauerstoffausschluß wird die Methanisierung in der Anaerob-Stufe begünstig bzw. nicht behindert.

Noch in der Flüssigkeit enthaltene Feststoffanteile, die in den Anaerob-Reaktor gelangen, werden von Zeit zu Zeit als Klärschlamm abgezogen, wobei hierüber auch zunächst gelöste und später in gebundenen Niederschlägen enthaltene geringe Metallmengen, insbesondere Schwermetallanteile, ausgeschieden werden können.

Im Anaerob-Reaktor wird bei der Behandlung mit mesophilen Bakterien eine Temperatur von 45°C bis 40°C, bei der Behandlung mit thermophilen Bakterien von 50°C bis 55°C während der gesamten Methanisierung aufrechterhalten. Das Befüllen des Anaerob-Reaktors mit dem Flüssigkeitsaustrag aus der Trennstufe erfolgt kontinuierlich oder im Abstand von 1 bis 2 Stunden. Die hydraulische Verweilzeit liegt bei ca. 8 bis 10 Tagen bei einer Abbaurate der organischen Substanz von 90 bis 95 %. Diese im Vergleich zu konventionellen Verfahren kurze Verweilzeit im Anaerob-Reaktor hat folgende Gründe.

Die zu methanisierende organische Substanz liegt nach dem erfindungsgemäßen Verfahren bereits in für die methanogenen Bakterien verwertbarer Form vor, ferner kann eine Ausschwemmung der Bakterien gezielt entgegengewirkt werden. Bei einer Abbaurate von mehr als 90 % verläßt nur gering belastetes Wasser den Anaerob-Reaktor. Der Gehalt an gelöster organischer Substanz im Wasser (DOC-Gehalt) kann durch entsprechende am Reaktor installierte Geräte automatisch überwacht werden. Ggf. kann das Wasser bei (noch) zu hohen DOC-Gehalten im Kreis geführt werden, d.h., es wird bis zur vollständigen Vergärung wieder in den Anaerob-Reaktor eingeleitet.

Zur Durchführung des erfindungsgemäßen Verfahrens wird die im Anspruch 9 beschriebene Vorrichtung vorgeschlagen. Diese besitzt einen Feststoffreaktor mit einer Mischeinrichtung und einem Flüssigkeitsaustrag und einen diesem nachgeschalteten Anaerob-Reaktor zur Vergärung in einem Biogas-Abzug oder eine Kompostiereinrichtung. Damit das oben anfallende Material aus biologischem Abfall nicht die weitere Schneckenförderung behindert, ist oberhalb und parallel der Schneckenpresse ein Tisch bzw. sind Tischbleche schrägliegend angeordnet. Hierauf kann das geförderte Material fallen und nach unten abrutschen, um dort angekommen abermals von der Schnecke erfaßt und nach oben gefördert zu werden. Je nach Zielsetzung, d.h., ob das Schwergewicht in die Biogasherstellung oder die Kompostherstellung gelegt wird, kann neben der Trennstufe alternativ oder nebeneinander ein Anaerob-Reaktor und/oder eine Kompostiereinrichtung nachgeschaltet sein. Derzeit liegen gewichtige Gründe vor, die Biogasherstellung in den Vordergrund zu stellen, da das Biogas energetisch verwertbar ist, während die Verwertung von Kompost sich auf wenige Einsatzbereiche beschränkt und kaum wirtschaftlich durchgeführt werden kann.

Weiterbildungen der Vorrichtung sind in den Ansprüchen 10 bis 17 beschrieben.

So besteht die Mischeinrichtung zur Beschleunigung des Abpressens bzw. Trennens der Feststoffe von den Flüssigstoffen, welche das Kohlenstoff in gelöster Form aufnehmen, aus einer Schneckenpresse, vorzugsweise aus zwei gegenläufigen schrägliegenden Schneckenpressen. Diese gegenläufig arbeitenden Schneckenwellen führen zu einer intensiven Durchmischung des biologischen Abfalls und gewährleisten eine gleichmäßige Sauerstoffversorgung und damit eine optimale mikrobielle Aktivität. Vorzugsweise arbeiten die Schneckenpressen ca. 5 bis 10 Minuten pro Stunde. Da die Schneckenpressen schrägliegend angeordnet sind, kann das Material von unten nach oben gefördert werden.

Nach einer weiteren Ausgestaltung der Erfindung sind der Tisch oder die Tischbleche als Wärmetauscher ausgebildet. Dies schafft deshalb Vorteile, weil das durch die Schneckenpresse geförderte Material jeweils mit dem Tisch in Berührung kommt und weil der Tisch relativ zentral in bezug auf den Materialstrom angeordnet werden kann.

Um zu vermeiden, daß Feststoffe größeren Durchmessers mit der Flüssigkeit ausgetragen werden, besitzt der Feststoffreaktor mindestens ein Sieb, vorzugsweise mehrere übereinanderliegende Spaltsiebe unterschiedlicher Maschenweite, die vor dem Flüssigkeitsaustrag angeordnet sind. Beispielsweise können drei übereinanderliegende Siebe verwendet werden.

Nach einer weiteren Ausgestaltung der Erfindung besitzt der Feststoffreaktor eine Gasabzugseinrichtung, die vorzugsweise mit einem Kompostfilter verbunden ist, wodurch eine Geruchsbelästigung der Außenumgebung vermeidbar ist.

Je nach Zusammensetzung des Bioabfalles, der in den Feststoffreaktor aufgegeben wird, kann es sich als die Trennung fördernd erweisen, wenn der Stellwinkel des Feststoffreaktors, d.h., insbesondere die Bodenneigung des Reaktors veränderbar ist.

Nach einer weiteren Ausgestaltung der Erfindung besitzt der Feststoffreaktor einen vorzugsweise verschließbaren Einfülltrichter und einen die Bio-Reststoffe fördernden Trogkettenförderer, der vorzugsweise gasdicht und/oder mit aktiver Luftabsaugung mittels Seitenkanalverdichtern ausgebildet ist. Die abgesaugte Luft kann ebenfalls über einen Kompostfilter zur Desodorierung gedrückt werden, um Geruchemissionen weitestgehend zu vermeiden.

Wie bereits oben erwähnt, ist vorzugsweise zwischen dem Feststoffreaktor und dem Anaerob-Reaktor ein Sedimentator geschaltet, der nach einer weiteren Ausgestaltung der Erfindung über einen Abzug verfügt, worüber die anfallenden Schlämme abgezogen und unmittelbar dem Feststoffreaktor wieder zuführbar sind. Insbesondere kann der Feststoffreaktor und/oder der Sedimentator über einen Belüftungskreislauf oder Luftzuführeinrichtungen verfügen, um den abgezogenen Schlamm zu einem Belebtschlamm aufzubereiten.

Um den Anaerob-Reaktor praktisch sauerstofffrei betreiben zu können, ist weiterhin zwischen dem Sedimentator und dem Anaerob-Reaktor ein Entgasungsreaktor, vorzugsweise ein Ultraschallreaktor oder Biostripper angeordnet.

Der Anaerob-Reaktor ist als Festbettreaktor ausgebildet und/oder weist mehrere Aufwuchs fördernde Gitterträger auf, die nach einer weiteren Ausgestaltung der Erfindung eine rauhe Oberfläche besitzen. Hierdurch wird eine Ausschwemmung der Bakterien wirksam verhindert, ferner die Aufwuchsgeschwindigkeit optimiert.

Im Anaerob-Reaktor setzen zunächst acetogene Bakterien die gelösten Zwischenprodukte in Acetat, Wasserstoff und Kohlendioxid um. Dies ist erforderlich, da Methanbakterien nur aus diesen Ausgangsprodukten Methan produzieren können. Im therminalen Schritt der Biogasproduktion wird durch methanogene Bakterien zum einen aus Wasserstoff und Kohlendioxid Methan und Wasser und zum anderen aus Acetat und Methan Kohlendioxid gebildet. Die erste Reaktion wird durch hydrogenotrophe Methanbakterien, die zweite durch acetotrophe katalysiert. Die Methanausbeute liegt bei ca. 70 Vol.-%. Ein limitierender Faktor bei der anaeroben Vergärung ist das relativ langsame Wachstum der Bakterien, d.h., eine Verdoppelung findet nur alle 14 Tage statt. Daher ist es notwendig, eine Stabilisierung bzw. eine Erhöhung der Mikroorganismenpopulation durch Aufwuchs auf festen Gitterträgern zu gewährleisten, wobei eine rauhe Gitteroberfläche die Aufwuchsgeschwindigkeit der Bakterien erhöht, unter Einhaltung eines für den Aufstieg der Gasblasen nötigen Lumens. Eine Verminderung der Bakterienpopulation durch Ausschwemmung kann jedenfalls erheblich minimiert werden, womit die Verweilzeit im Anaerob-Reaktor wesentlich verkürzt bzw. das Reaktorvolumen entsprechend klein gewählt werden kann.

Nach einer weiteren Ausgestaltung der Erfindung weist der Anaerob-Reaktor einen Gasdom auf, der vorzugsweise mit einem Gasabscheider versehen ist, wohin es mit geringem Unterdruck weitergeleitet wird.

Der Anaerob-Reaktor kann als Methanisierungsstufe aus mehreren hintereinandergeschalteten und segmentrierten Festbettreaktoren bestehen, die über kommunizierende Röhren miteinander verbunden sind. Der Transport des Wassers erfolgt dann nach dem Prinzip der kommunizierenden Röhren allein über die Zufuhr in den ersten Festbettreaktor. Beispielsweise können die Festbettreaktoren zylindrische Röhren von 4 m Höhe und 1,2 m Durchmesser sein, die jeweils im downflow-Modus beschickt werden. Dem letzten Festbettreaktor kann ein Sedimentationsgefäß nachgeschaltet sein.

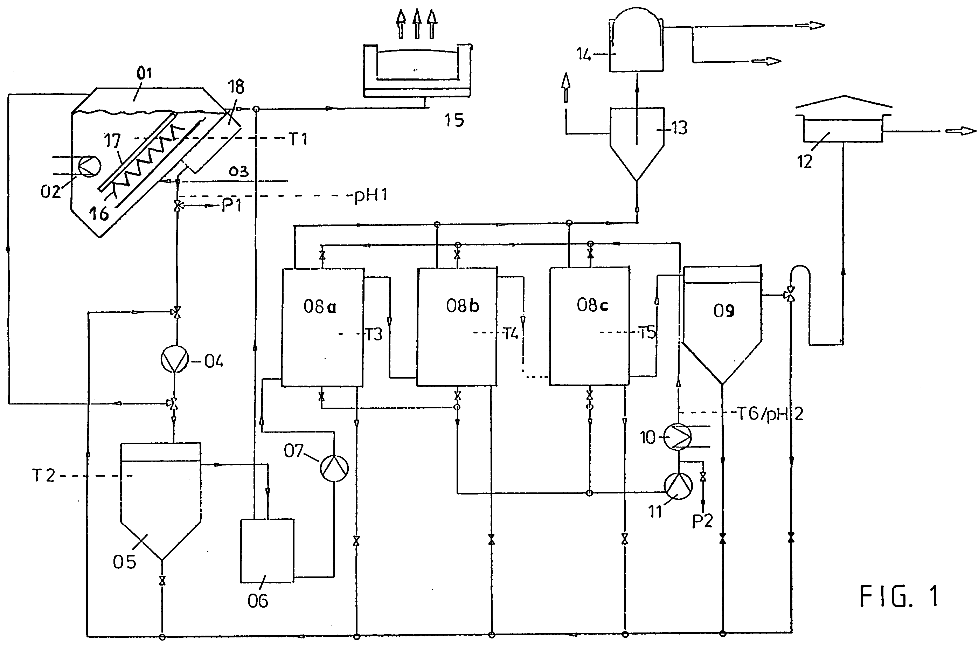

Ein Ausführungsbeispiel der Erfindung ist in den Zeichnungen dargestellt. Es zeigen
- Fig. 1: eine schematische Übersicht der erfindungsgemäßen Vorrichtung,
- Fig. 2: eine schematische Seitenansicht des Feststoffreaktors,
- Fig. 3: eine erste Querschnittsansicht des Schneckenförderers in Höhe der Leitblech-Anordnung,
- Fig. 4: eine weitere Ansicht des Schneckenförderers in Höhe des Spaltsiebes,
- Fig. 5: eine schematische Seitenansicht des Trogkettenförderers mit dem Festbettreaktor,
- Fig. 6: eine Draufsicht auf die Anordnung nach Fig. 5 und
- Fig. 7: ein Fließschema des erfindungsgemäßen Verfahrens.

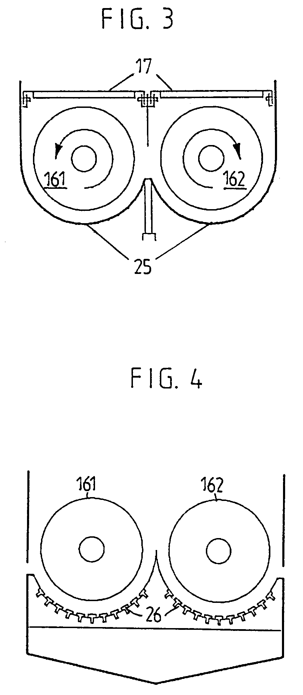

Die wesentlichsten Teile der Vorrichtung sind der Feststoffreaktor 01 sowie der aus drei Teilen bestehende Anaerob-Reaktor 08a, b und c, die das Kernstück der gesamten Anlage darstellen. Die zu behandelnden biologischen Rest- oder Abfallstoffe werden in den Feststoffreaktor 01 gegeben, in dem sie von einer Schneckenwelle 16 oder zwei gegenläufig arbeitenden Schneckenwellen (siehe Fig. 3, 4) im unteren Teil des Feststoffreaktors 01 erfaßt und nach oben gefördert werden. Im oberen Bereich, d.h. oberhalb der Preßzone mit einem Spaltsieb, wird die Feuchtigkeit aus den Abfallstoffen ausgepreßt und über das Spaltsieb aus dem Reaktor abgepreßt. Oberhalb und parallel zu der Schnecke oder den gegenläufigen Schneckenwellen ist ein Tisch bzw. sind Tischbleche 17 angeordnet, die als Wärmetauscher 02 ausgebiledet sind. Der Effekt des Auspressens der biologischen Abfallstoffe kann noch durch den Gegendruck der Tischbleche verstärkt werden sowie durch eine sich zwischen dem Spaltsieb und der Schneckenwelle zum Austrag hin verjüngende Distanz. Ggf. kann diese Distanz auch durch die Verstellung des Drehpunktes an der Abpreß- bzw. Entleerungsvorrichtung 18 verändert werden. Soweit die Spaltbreite des Siebes nicht am Sieb veränderbar ist, sind die Siebe auswechselbar und somit an die jeweiligen Anforderungen der zu verarbeitenden biologischen Reststoffe anpaßtbar. Die von der überschüssigen Feuchtigkeit befreiten Abfallstoffe werden durch das kontinuierlich nachgeförderte Material aus der Sieb- bzw. Preßzone in den freien Reaktorraum gedrückt. Hierbei rutschen sie schwerkraftbedingt über das Tischblech wieder an die tiefste Stelle des Feststoffreaktors 01 zurück, wie sie erneut von den Schneckenwellen 16 erfaßt werden. Auf diese Art und Weise können die im Kreislauf umgewälzten Abfallstoffe kontinuierlich von der überschüssigen Feuchtigkeit befreit bzw. die Kohlenstoffgehalte der Abfallstoffe durch vorhandene Flüssigkeit ausgewaschen werden. Der Feststoffreaktor besitzt eine gesteuerte Sauerstoffzufuhr 03, um eine optimale mikrobielle Aktivität zu gewährleisten. Die Sauerstoffzufuhr kann über Sauerstoffgehaltsmessungen oder CO₂-Konzentrationsmessungen gesteuert bzw. geregelt werden. Während der im Feststoffreaktor 01 stattfindenden aeroben Intensivrotte werden energiereiche polymere Substanzen, wie Fette, Proteine und Kohlenhydrate in energieärmere, monomere Bestandteile, wie Fettsäuren, Aminosäuren und Zucker, zerlegt. Die Dauer der im Feststoffreaktor 01 stattfindenden vorgeschalteten aeroben Intensivrotte ist variabel und von der Zielsetzung abhängig, ob mehr Biogas oder mehr Kompost produziert werden soll. Beispielsweise können während einer 48-stündigen Aufenthaltszeit im Feststoffreaktor 01 bereits die leicht und mittelschwer abbaubaren organischen Substanzen soweit in niederkettige Fettsäuren und Alkohole gespalten werden, daß sie anaerob im Anaerob-Reaktor 08a, b und c weiter zum hauptsächlichen Endprodukt Methan und Kohlendioxid durch acetogene und methanogene Bakterien abgebaut werden können. Die Fettsäuren und Alkohole sind in bei der Umsetzung austretendem Wasser, das durch die natürliche Feuchte vorhanden ist oder im Zellwasser gebunden und ausgepreßt wird, gelöst und suspendiert. Die Suspension mit hohen Anteilen an aerob hydrolisierten organischen Substanzen wird über das bereits genannte Sieb von der Masse im Feststoffreaktor abgetrennt. Beim Auslaugen der löslichen organischen Bestandteile zusammen mit der aeroben Intensivrotte findet eine starke Volumenreduktion (70 bis 80 Vol-%) des Materials statt. Die aus den biologischen Abfällen gewonnene Suspension wird über ein Ventil P1 durch die Pumpe 04 in einen Sedimentator 05 gefördert. Die dort abgetrennten Feststoffe, die sich als Schlamm niederschlagen, können zum Beimpfen des frisch eingefüllten biologischen Abfalles wieder in den Feststoffreaktor 01 gepumpt werden. Das feststofffreie, mit organischen Inhaltsstoffen, insbesondere Kohlenstoffanteilen, angereicherte Wasser wird in den Anaerob-Reaktor 08 gepumpt. Die restliche, ausgewaschene Feststoffmasse wird als Frischkompost mit einem Rottegrad von I und II einer Nachrotte in Form einer Mietenkompostierung unterworfen. Der Frischkompost wird abgefahren.

Das Befüllen des Feststoffreaktors erfolgt über eine verschließbare Öffnung im Feststoffreaktoroberteil, die nur wahrend des Befüllens geöffnet wird. Das Ausschleusen des Frischkompostes erfolgt über eine elektromotorisch betriebene Austragsschleuse. Das Material fällt in einen verschließbaren Container (nicht dargestellt).

Zur Vermeidung von Geruchsemissionen ist der Feststoffreaktor über eine Gasleitung mit einem Kompostfilter 15 verbunden. Während des Betriebes bleibt der Feststoffreaktor vorzugsweise gasdicht verschlossen. Eingeblasene Luft zur Erzeugung aerober Verhältnisse verläßt den Reaktor nur durch das Kompostfilter. Wird der Reaktor zur Befüllung geöffnet, beginnt ein in der Gasleitung montierter Seitenkanalverdichter automatisch Luft vom Reaktor in das Kompostfilter zu saugen. Es entsteht hierbei eine invers gerichtete Luftströmung, so daß eine Geruchemission aus dem Feststoffreaktor 01 nicht entstehen kann.

Um sicherzustellen, daß das von Feststoffen befreite bzw. die stark feststoffreduzierte Suspension möglichst keinen Sauerstoff in den Anaerob-Reaktor 08 einbringt, ist eine Ultraschallentgasungseinrichtung 06 vorgesehen, durch die die Flüssigkeit geleitet und mittels der Pumpe 7 schließlich in den Anaerob-Reaktor 08 geführt wird. Der mehrstufige Anaerob-Reaktor 08a, b und c, der mittels kommunizierender Röhren im Kreislauf betreibbar ist, ist als Festbettreaktor ausgerüstet. Dem Anaerob-Reaktor 08 ist ein weiterer Sedimentator 09 nachgeschaltet, durch den das noch mit geringen Feststoffanteilen belastete Wasser nach der Methanisierung geführt und von Feststoffen befreit wird. Das Wasser kann anschließend rezyklierend in den Kreislauf zurückgeführt werden. Der Anaerob-Reaktor besitzt einen Wärmetauscher 10, der, vorzugsweise über eine entsprechende Regelung, mit dem Wärmetauscher 02 in Verbindung steht, so daß die bei der exothermen Reaktion im Festbettreaktor 01 anfallende Wärme in den Anaerob-Reaktor 08 geführt werden kann. Die Pumpe 11 unterstützt eine etwa notwendige Kreislaufförderung der Flüssigkeit durch die Stufe 08a, b und c.

Das den Anaerob-Reaktor 08 verlassende Wasser, mit einem DOC-Gehalt von 1 g/l und einem CSB-Gehalt von ca. 2 bis 3 g/l, wird in eine Pflanzenkläranlage 12 geleitet und von dort bis zur Vorfluterqualität gereinigt. Hierbei handelt es sich um eine mit Helophyten besetzte, vertikal durchströmte, mehrschichtige Festbettfilteranlage mit selbsttätigem Intervallbetrieb. Dieser Festbettfilter ist durch die Abfolge von aerober Bodenschicht mit einem Durchlässigkeitsbeiwert (Kf) von 5 x 10⁻³ m/s charakterisiert. In dieser Schicht kommt es zur Nitrifizierung des Stickstoffes und vor allem zur Oxidation der im Wasser gelösten restlichen organischen Substanz. Die darauffolgende dichte Bodenschicht mit einem Kf-Wert von 10⁻⁷ m/s begünstigt eine Denitrifikation des Stickstoffes, welcher gasförmig als N₂ durch eine entsprechende Leitung in die Atmosphäre entweichen kann. Durch den höheren Feinkornanteil wird ebenfalls ein hohes Phosphatbindungspotential sichergestellt. Die Durchlässigkeit der unteren Filterschicht entspricht der oberen. Es kommt zu einer Restmineralisation der organischen Substanz, Nitrifikation vorhandener N-Komponenten und zu einer weiteren Keimzahlreduktion.

Versuche haben erwiesen, daß dieses vertikal durchströmte System der Pflanzenkläranlage, die an Wasser mit Vorfluterqualität gestellten Kriterien einhält, diese sogar unterschreitet.

Über das Wurzelsystem des Schiffes (Rhizom) wird die Sickerleistung ständig gewährleistet. Die Halme beschatten den Bodenkörper und verhindern eine unerwünschte Algenbildung. Sie bilden ein günstiges Mikroklima für Mikroorganismen. Eine wichtige Eigenschaft der Pflanzen besteht in der Fähigkeit, über ein spezielles Luftleitgewebe (Aerenchym) Sauerstoff in den Boden einzutragen. Die Anlage ist auf einer Fläche von 6 m x 5 m untergebracht. Der Wasserabfluß der Anlage wird aufgefangen und über eine Woche gehältert. Erst nach Analyse des Wassers auf die Parameter nach Indirekteinleiterverordnung wird über die Einleitung in die Vorflut oder den Transport in eine Kläranlage entschieden.

Das im Anaerob-Reaktor entstehende Biogas gelangt über einen Gasabscheider 13 in den Gasspeicher 14, der zur Pufferung von Mengenschwankungen bei der Biogaserzeugung dient, um eine gleichmäßige Füllung eines nachgeschalteten Gasmotors aufrechtzuerhalten. Hier kann ein Sterling-Motor eingesetzt werden.

Der Kompostfilter 15 der aus jeder Prozeßstufe gelangende geruchsbeladene Abluftströme aufnehmen kann, besteht vorzugsweise aus einer in 2,5 m-Schachtringen, bis zur Oberkante in den Boden eingelassenen Spezialkompostschicht. Das zu desodorierende Gas wird von der Unterseite her durch das Filter geleitet. Es durchströmt die Kompostschichten. Geruchsaktive Substanzen ad- und absorbieren und werden durch die Mikroorganismen verstoffwechselt. Das den Filter verlassende Gas ist normalerweise annähernd geruchsneutrag oder weist einen leicht erdigen Geruch auf, der jedoch nur direkt oberhalb des Filters wahrnehmbar ist. Die Abluft ist frei von möglichen pathogenen Keimen, die ggf. über den Abfall in den Feststoffreaktor 01 eingetragen werden.

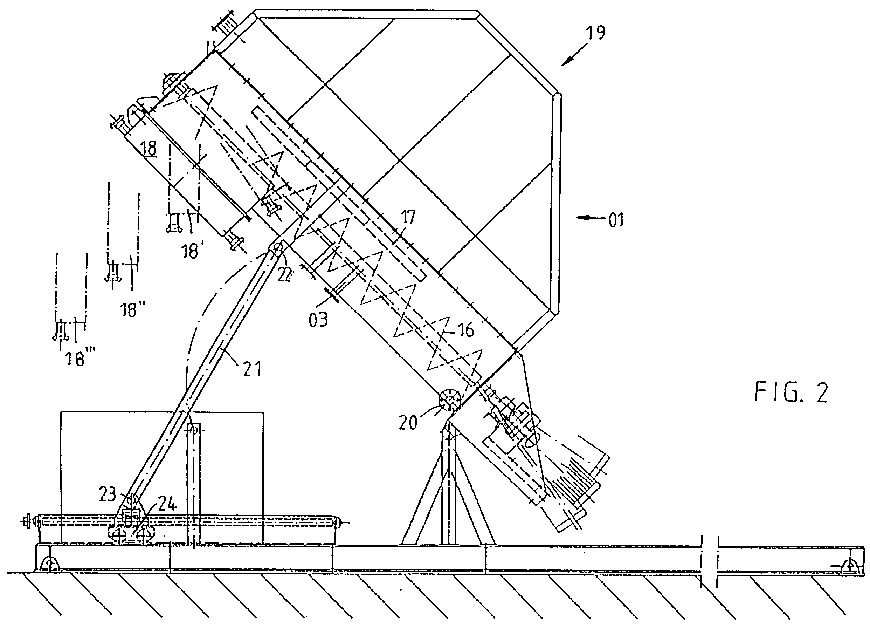

Aus Fig. 2 bis Fig. 4 sind Details des Feststoffreaktors ersichtlich. Der Feststoffreaktor besitzt eine obere Füllöffnung 19, die nur zum Befüllen geöffnet und im übrigen Betrieb möglichst gasdicht verschlossen ist. Der Feststoffreaktor 01 ist an einer ersten Drehachse 20 angelenkt und über einen Schwenkarm 21 einseitig derart heb- und senkbar, daß unterschiedliche Winkelneigungen eingestellt werden können. Der Schwenkarm 21 ist einerseits über ein Gelenk 22 mit dem Feststoffreaktor 01 und andererseits über ein weiteres Gelenk 23 mit einem Rollwagen 24 verbunden, der linear verfahrbar ist. Zur Durchmischung und Homogenisierung besitzt der Feststoffreaktor mindestens eine Schneckenwelle 16, vorzugsweise zwei gegenläufige Schneckenwellen 161 und 162, wie dies aus Fig. 3 und 4 ersichtlich ist. Oberhalb der Schneckenwellen ist ein als Wärmetauscher 02 ausgebildeter Tisch 17 angeordnet. Im oberen Bereich besitzt der Feststoffreaktor eine Abpreß- und Entleerungsvorrichtung 18, die etwa in die Positionen 18', 18'' und 18''' geschwenkt werden kann und ein Entladen des Feststoffreaktors ermöglicht, falls der biologische Abfall vollständig ausgewaschen und abgepreßt ist.

Die Schneckenwellen 161 und 162 sind in Halbschalen 25 angeordnet (Fig. 3).

Im oberen Bereich der Entleerungsvorrichtung 18, also etwa im oberen Drittel des Feststoffreaktorbodens ist ein Spaltsieb 26 in den Halbschalen vorgesehen, wobei die Spaltöffnungen parallel zur Förderrichtung laufen und/oder das Spaltsieb gegenüber dem Umfang der sich drehenden Schneckenwelle ein sich verjüngenden Abstand aufweisen kann. Vorzugsweise ist der Abstand zwischen dem Spaltsieb und der Förderschnecke sowie der Anpreßdruck veränderbar.

Es kann auch vorgesehen sein, daß die Förderrichtung durch Änderung der Schneckenwindung oberhalb der Preßzone wechselt und hierdurch eine erhöhte Preßwirkung erzielt wird.

Ggf. besitzt der Feststoffreaktor an seinem tiefsten Punkt noch eine wiederverschließbare Flüssigkeits- bzw. Suspensionsauslaßöffnung.

Um Verstopfungen im Förderbereich zu vermeiden, können flexible Vorrichtungen, wie Bürsten, Gummibänder oder auch Abstreiffinger und ähnliches, vorgesehen sein, die die Förderschnecke bzw. Schneckenwellen jeweils reinigen.

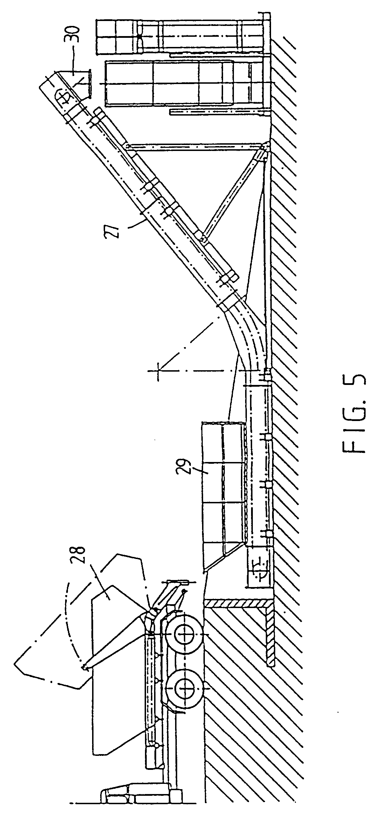

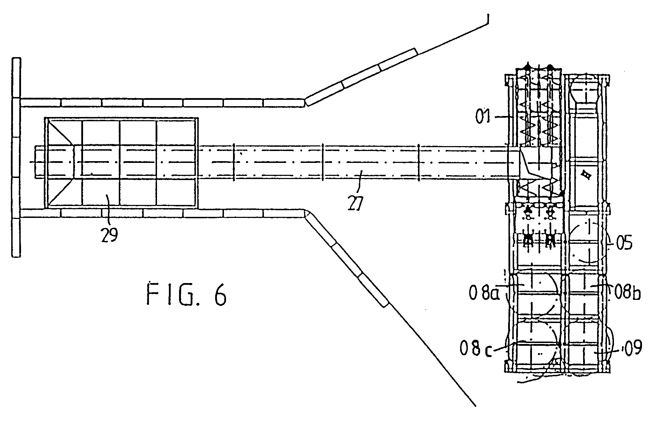

Fig. 5 und 6 zeigen eine zweckmäßige Erweiterung der bisher dargestellten Vorrichtung. Dem Feststoffreaktor 01 ist zweckmäßigerweise ein Trogkettenförderer 27 vorgeschaltet. Der Trogkettenförderer wird durch ein Containerfahrzeug 28 beladen, das den biologischen Abfall in einen Aufgabetrichter 29 transportiert. Der Abfall wird in einem geschlossenen Förderkasten zum Austrag 30 transportiert, der sich über der Einfüllöffnung 19 des Feststoffreaktors 01 befindet. Überschüssiges Material, das nicht direkt in den Feststoffreaktor 01 eingebracht werden kann, verbleibt bis zu nächstmöglichen Befüllung im Förderer. Der Einfülltrichter 29 ist zur Vermeidung von Geruchsemissionen nur während des Befüllvorganges geöffnet. Außerhalb dieser Zeiten ist das System mittels verspannter Plane geschlossen. Der Austrag des Förderers ist mittels einer beweglichen Platte oder eines Schiebers ebenfalls verschlossen; der Fördertrog ist ebenfalls gasdicht. Ggf. kann der Trogkettenförderer mit einer aktiven Luftabsaugung versehen sein, die während des Betriebes sowie außerhalb des Befüllvorganges in festlegbaren Intervallen aus dem Förderer mittels eines Seitenkanalverdichters Luft absaugt und diese dem Kompostfilter 15 zur Desodorierung zuführt.

Der Einfülltrichter 29 des Trogkettenförderers 27 sowie der Förderer selbst stellen das Zwischenlager für den angelieferten biologischen Abfall dar. Anfallendes Sickerwasser wird nach im Förderer gefaßt und in den Feststoffreaktor 01 oder den nachgeschalteten Sedimentator 05 geleitet.

Wie Fig. 6 zu entnehmen, ist in der dargestellten Ausführungsform der Feststoffreaktor 01 in der Draufsicht langgestreckt ausgeführt. In unmittelbarer Nachbarschaft des Feststoffreaktors 01 befinden sich der Sedimentator, mit einem Gasvolumen von etwa 3 m³ sowie der Anaerob-Reaktor 08, bestehend aus drei hintereinandergeschalteten und segmentierten Festbettreaktoren 08a, 08b und 08c mit jeweils 4 m³ Kammervolumen in zylindrischen Röhren von 4 m Höhe und 1,2 m Durchmesser. Insgesamt können die Festbettreaktoren 08a, b und c mit einer Raumbelastung bis zu 35 kg OTS/m³xd gefahren werden. Den drei Reaktoren 08a, b und c ist eine vierte Röhre als Sedimentationsgefäß 09 nachgeschaltet. Die Röhren 08a, b und c und 09 bilden eine Einheit und sind auf einer Grundfläche von 2,7 m x 2,7 m installiert und insgesamt isoliert.

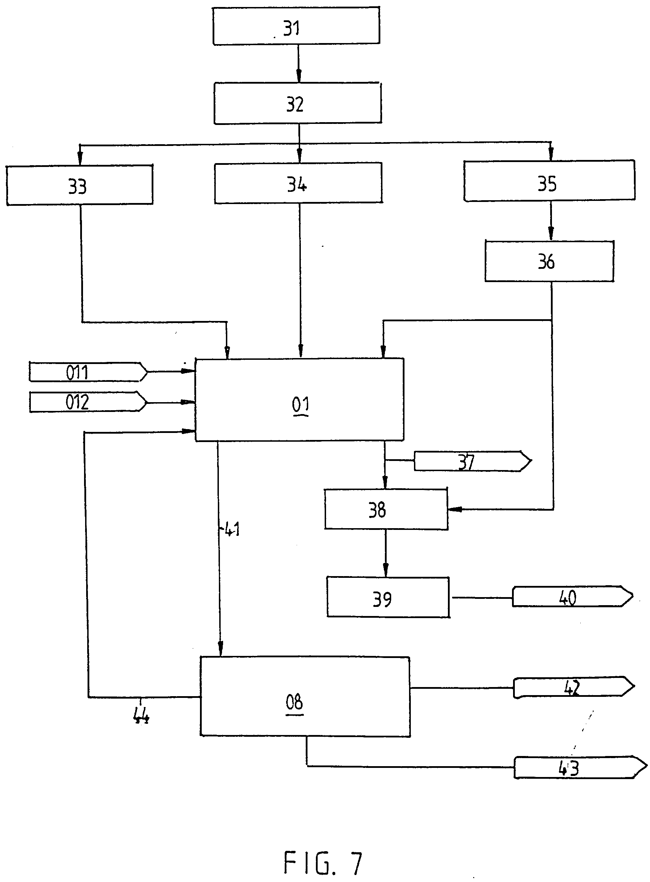

Mit der beschriebenen Vorrichtung wird das erfindungsgemäße Verfahren, wie aus dem Fließschema nach Fig. 7 zu ersehen, durchgeführt. Nach Anlieferung 31 und Qualitätskontrolle 32 der angelieferten biologischen Abfälle, bestehend aus gewerblichen kommunalen Abfällen 33, strukturarmen nassen Reststoffen 34 und strukturreichen trockenen Reststoffen 35, die zunächst einer Zerkleinerung 36 zugeführt werden, werden die jeweiligen Abfälle einzeln oder in vorbestimmbaren Mischungen dem Feststoffreaktor 01 zugeführt, eventuelle unter Zugabe von Luft 011 und/oder sonstigen Additiven 012. Die aus dem Feststoffreaktor nach Entwässerung verbleibenden Feststoffe werden entweder als Frischkompost 37 oder nach Mischen 38 einer Nachverrottung 39 zugeführt, wo sie zu einem Reifekompost 40 umgewandelt werden. Über einen Austrag 41 werden die abgepreßten Flüssiganteile nach Sedimentierung dem Anaerobfilter 08 zur anaeroben Naßvergärung zugeführt, woraus die Biogase 42, im wesentlichen Methan, sowie das zurückbleibende Wasser 43 abgeführt werden. Teile der Flüssigkeitsmenge können auch als Rezirkulat 44 dem Feststoffreaktor 01 zurückgeführt werden.

In einer durchgeführten Versuchscharge sind 2 Tonnen der Abfallstoffe 33, 34 und 35 aus Haushaltsabfällen im Feststoffreaktor 01 durchmischt und unter Lufteintrag behandelt worden, wobei eine Temperatur von 72°C erreicht wurde. Die während der Umsetzung anfallende organische Substanz im Preßwasser 14 betrug ca. 23 % der ursprünglichen Einwaage. Durch die Vergärung in dem Anaerob-Reaktor 08 bildete sich aus diesen organischen Substanzen ca. 210 m³ Biogas 42 mit einem Methangehalt von 65 Vol.-%. Der aktive Kompost wurde als Feststoffaustrag mit gehäckseltem Strukturmaterial, stehend aus Rinde und Strauchschnitt, etwa im Verhältnis 1 : 1 gemischt und drei Wochen auf einer Miete ohne Umsetzung liegengelassen. Hiernach wies der Kompost einen erdigen Geruch auf und war intensiv mit einer Pilzkultur belegt. Der erhaltene Kompost erfüllte die vorgegebenen Grenzwerte.

## Patentansprüche

1. Verfahren zur Behandlung von organischen Bio-Reststoffen (31, 33 bis 35), insbesondere aus kommunalen und/oder gewerblichen Abfallstoffen (33) einschließlich roher und/oder gekochter Speisereste (34), landwirtschaftliche Abfallstoffe, insbesondere Tierexkremente und/oder pflanzlicher Bestandteile, bei dem die Bio-Reststoffe (31, 33 bis 35) in einen Feststoff- und einen Flüssig-Anteil getrennt werden und anschließend der Feststoffanteil einer aeroben Mietenkompostierung und der Flüssig-Anteil einem Anaerob-Reaktor zugeführt wird, von dem das durch Vergärung entstandene Biogas abgezogen wird,
**gekennzeichnet durch**
daß aus den Bio-Reststoffen (31, 33 bis 35) vor der Fest-Flüssig-Trennung unter intensivem Mischen und Homogenisieren die in Wasser gelösten und lösbaren C-haltigen (organischen) Bestandteile (DOC) ausgewaschen werden.

2. Verfahren nach Anspruch 1, dadurch gekennzeichnet, daß in Trennstufe a) die Ausgangsfeuchte oder die Feuchte von 70 bis 80 Vol.-%, zumindest jedoch 40 bis 60 Vol.-%, während des Auswaschens von in Wasser gelösten organischen Bestandteilen, ggf. durch Zuführung von Wasser (44) aufrechterhalten wird, wobei vorzugsweise das durch erfolgte Methanisierung von C-haltigen Bestandteilen befreite Wasser (44) aus dem Anaerob-Reaktor (08) rezyklierend in die Trennstufe a) gegeben wird.

3. Verfahren nach einem der Ansprüche 1 oder 2, dadurch gekennzeichnet, daß in der Trennstufe a) gezielt Sauerstoff (011) zugeführt wird und/oder eine Temperatur von bis zu 70°C konstant aufrechterhalten wird, ggf. mit der anschließenden Maßnahme, abschließend Wärme zur Erzeugung einer Temperatur von 80°C bis 90°C zuzuführen, um eine möglichst vollständige Hygienisierung durchzuführen und/oder daß die bei den exotherm verlaufenden Reaktionen in der Trennstufe entstehende Wärme abgezogen und in den Anaerob-Reaktor (08) geleitet wird.

4. Verfahren nach einem der Ansprüche 1 bis 3, dadurch gekennzeichnet, daß erst nach Unterschreiten eines vorgebbaren Gehaltes an gelösten C-haltigen Bestandteilen in der Flüssigkeit (41) die verbleibenden Reststoffe auf einen möglichst niedrigen Restfeuchtigkeitsgehalt abgepreßt werden, bevor die gesamte Charge an Reststoffen aus der Trennstufe abgeführt und anschließend kompostiert wird, wobei die Trennung von Flüssigbestandteilen (41) und Reststoffen (37, 39) vorzugsweise durch Pressen und/oder Sieben erfolgt.

5. Verfahren nach einem der Ansprüche 1 bis 4, dadurch gekennzeichnet, daß die als Suspension vorliegende C-haltige Bestandteile enthaltene Flüssigkeit (41) sedimentiert wird, bevor sie dem Anaerob-Reaktor (08) zugeführt wird, wobei vorzugsweise der beim Sedimentieren als Schlamm anfallende Feststoff als Animpfmaterial zur Reaktionsbeschleunigung, vorzugsweise nach Sauerstoffanreicherung nach frischer Füllung den Bio-Reststoffen zugeführt wird.

6. Verfahren nach einem der Ansprüche 1 bis 5, dadurch gekennzeichnet, daß die aus der Trennstufe abgeführte Flüssigkeit (41) vor der Einleitung in den Anaerob-Reaktor (08) einer Ultraschallbehandlung oder Biogasstrippung zur Entfernung noch enthaltenden Sauerstoffes unterzogen wird und/oder daß in der Flüssigkeit (Suspension) noch enthaltende Feststoffanteile von Zeit zu Zeit als Klärschlamm aus dem Anaerob-Reaktor (08) ggf. mit zunächst gelösten und später in gebundener Form in Niederschlägen enthaltenen geringen Metall-, insbesondere Schwermetallanteile abgezogen werden.

7. Verfahren nach einem der Ansprüche 1 bis 6, dadurch gekennzeichnet, daß im Anaerob-Reaktor (08) bei der Behandlung mit mesophilen Bakterien eine Temperatur von 45 bis 40°C, bei der Behandlung mit thermophilen Bakterien von 50 bis 55°C aufrechterhalten wird.

8. Verfahren nach einem der Ansprüche 1 bis 7, dadurch gekennzeichnet, daß der Anaerob-Reaktor (08) mit dem Flüssigkeitsaustrag (41) aus der Trennstufe kontinuierlich oder im Abstand von 1 bis 2 h befüllt wird.

9. Vorrichtung zur Gewinnung von Biogas (42) und Kompost (37, 40) durch Naßvergärung und Kompostieren von organischen Bio-Reststoffen (31, 33 bis 35) mit einem als Preßschnecken-Fest-Flüssig-Separator ausgebildeten Feststoffreaktor (01), der einen Flüssigkeitsaustrag und einen diesem nachgeschalteten Anaerob-Reaktor (08) zur Vergärung mit einem Biogasabzug und eine Kompostiereinrichtung besitzt, dadurch gekennzeichnet, daß der Feststoffreaktor (01) eine schrägliegend angeordnete Schneckenpresse (16) aufweist, daß oberhalb und parallel dieser Schneckenpresse (16) ein Tisch oder Tischbleche (17) angeordnet sind und daß der Feststoffreaktor (01) ein Ventil (P1) zur Sedimententnahme aufweist.

10. Vorrichtung nach Anspruch 9, gekennzeichnet durch zwei gegenläufige schrägliegende Schneckenpressen (161, 162) und/oder als Wärmetauscher (02) ausgebildeten Tisch oder Tischbleche (17).

11. Vorrichtung nach einem der Ansprüche 9 oder 10, dadurch gekennzeichnet, daß der Feststoffreaktor (01) über mindestens ein Sieb (26), vorzugsweise mehrere übereinanderliegende Spaltsiebe unterschiedlicher Maschenweite vor dem Flüssigkeitaustrag und/oder über eine Gasabzugseinrichtung verfügt, die vorzugsweise mit einem Kompostfilter (15) verbunden ist.

12. Vorrichtung nach einem der Ansprüche 9 bis 11, dadurch gekennzeichnet, daß die im oberen Drittel des Reaktorbodens angeordnete Schneckenpresse (16; 161, 162) mit einem auswechselbaren Spaltsieb (26) ausgerüstet ist, dessen Spaltöffnungen parallel zur Förderrichtung laufen, und/oder das das Spaltsieb gegenüber dem Umfang der sich drehenden Schneckenwelle (16; 161, 162) einen sich verjüngenden Abstand aufweist, wobei vorzugsweise der Abstand zwischen dem Spaltsieb (26) und der Förderschnecke (16; 161, 162) und der Anpreßdruck durch eine verstellbare Aufhängung der Schneckenpresse verändert werden kann.

13. Vorrichtung nach Anspruch 12, dadurch gekennzeichnet, daß das durch die Schneckenpresse (16; 161, 162) freigesetzte Wasser in eine mit einer Spülvorrichtung versehene Auffangwanne (25) mit schägem Boden aufgefangen und abgeleitet wird und/oder daß die Förderrichtung durch Änderung der Schneckenwindung oberhalb der Preßzone wechselt und dadurch eine erhöhte Preßwirkung entsteht, wobei vorzugsweise am tiefsten Punkt des Reaktorbodens ebenfalls eine wahlweise verschließbare Öffnung zur Entnahme von Preßwasser angeordnet ist.

14. Vorrichtung nach einem der Ansprüche 9 bis 13, gekennzeichnet durch flexible Vorrichtungen, wie Bürsten, Gummibänder etc. am Rand der Schneckenwellen (16; 161, 162) zur Reinigung und Vorbeugung von Verstopfungen der Spaltsiebund Entleerungsöffnungen und/oder durch einen veränderbaren Stellwinkel des Feststoffreaktors (01), insbesondere der Bodenneigung.

15. Vorrichtung nach einem der Ansprüche 9 bis 14, dadurch gekennzeichnet, daß der Feststoffreaktor (01) einen vorzugsweise verschließbaren Einfülltrichter (19) und einen die Bio-Reststoffe fördernden Trogkettenförderer (27) aufweist, der vorzugsweise gasdicht und/oder mit aktiver Luftabsaugung mittels Seitenkanalverdichter ausgebildet ist, wobei vorzugsweise zwischen dem Feststoffreaktor (01) und dem Anaerob-Reaktor (08) ein Sedimentator (05) geschaltet ist, der vorzugsweise über einen Abzug verfügt, über den die anfallenden Schlämme abgezogen und unmittelbar dem Feststoffreaktor (01) wieder zuführbar sind und/oder wobei der Feststoffreaktor (01) und/oder der Sedimentator (05) über einen Belüftungskreislauf oder Luftzuführeinrichtungen (03) verfügen.

16. Vorrichtung nach einem der Ansprüche 9 bis 15, dadurch gekennzeichnet, daß zwischen Sedimentator (05) und dem Anaerob-Reaktor (08) ein Entgasungsreaktor, vorzugsweise Ultraschallreaktor (06) oder Biostripper angeordnet ist und/oder daß der Anaerob-Reaktor (08) als Festbettreaktor ausgebildet ist und/oder mehrere Aufwuchs fördernde Gitterträger aufweist, die vorzugsweise eine rauhe Oberfläche besitzen.

17. Vorrichtung nach einem der Ansprüche 9 bis 16, dadurch gekennzeichnet, daß der Anaerob-Reaktor (08) einen Gasdom, vorzugsweise mit einem Gasabscheider aufweist und/oder daß mehrere Festbettreaktoren (08a, b, c) vorgesehen sind, die über kommunizierende Röhren miteinander in Verbindung stehen.

## Claims

1. Process for treating organic bio-residues (31, 33 to 35), particularly from communal and/or industrial waste (33) including raw and cooked food leftovers (34), agricultural waste, particularly animal excrements and/or plant components, wherein the bio-residuals (31, 33 to 35) are separated in a solid and a liquid fraction and subsequently the solid fraction is fed to an aerobic stack composting and the liquid fraction is fed to an anaerobic reactor from which the biogas generated during fermentation is extracted,
characterized in that the C-containing (organic) components dissolved or soluble in water (DOC) are washed out from the bio-residues (31, 33 to 35) prior to the solid-liquid separation, under intensive mixing and homogenisation.

2. Process according to claim 1, characterized in that in the separation stage a) the initial humidity or the humidity of 70 to 80 % by volume, but at least 40 to 60 % by volume, is maintained while the organic components dissolved in water are being washed out, optionally by supply of water (44), whereby preferably the water (44) released through the performed methanisation of C-containing components is returned from the anaerobic reactor (8) to the separating stage a) in a recycling manner.

3. Process according to one of claims 1 to 2, characterized in that the separating stage a) oxygen (011) is supplied in a controlled manner and/or a temperature of up to 70°C is constantly maintained, optionally with the subsequent step to supply heat for producing a final temperature of 80°C to 90°C, in order to perform a hygienisation as complete as possible and/or that the heat resulting during the exothermic reaction in the separating stage is extracted and directed into the anaerobic reactor (08).

4. Process according to one of claims 1 to 3, characterized in that only after the content of C-containing components in the liquid (41) has fallen below a predetermined level, the remaining residual matter is squeezed out to possible lowest residual wetness content prior to the discharge of the entire charge of residues from the separation stage and subsequently composted, whereby the separation of liquid components (41) and residual materials (37, 39) is accomplished by squeezing and/or sieving.

5. Process according to one of claims 1 to 4, characterized in that the C-containing components contained in the liquid (41) as a suspension are subjected to sedimentation prior to being delivered to the anaerobic reactor (8), whereby preferably the solid materials resulting as sludge during sedimentation is introduced in the biological residues as inoculum for reaction acceleration, preferably after enrichment with oxygen after a fresh filling.

6. Process according to one of claims 1 to 5, characterized in that the liquid (41) evacuated from the separation stage prior to introduction in the anaerobic reactor (08) is subjected to ultrasound treatment or biogas stripping in order to remove the oxygen which it may still contain and/or the solid particles still contained in the liquid (suspension) are removed from time to time as slurry from the anaerobic reactor (08), optionally together with small amounts of metals, particularly heavy metals, thereby eliminating at first dissolved and later in combined precipitates.

7. Process according to one of claims 1 to 6, characterized in that the anaerobic reactor (08) during the treatment with mesophilic bacteria a temperature of 45 to 40°C is maintained, while in the treatment with thermophilic bacteria 50 to 55°C are maintained.

8. Process according to one of claims 1 to 7, characterized in that the anaerobic reactor (08) is continuously supplied with the liquid discharge (41) from the separation stage at intervals of 1 to 2 h.

9. Device for the production of biogas (42) and compost (37, 40) through wet fermentation and composting of bio-residuals (31, 33 to 35) with a reactor (01) for solid materials built worm-press type solid-liquid separator, which has a liquid outlet and a subsequently arranged anaerobic reactor (08) for fermentation with biogas extraction and a composting installation, characterized in that the reactor (01) for solid materials has a worm press (16) arranged at an angle, that above and parallel to this worm press (16) a table or table plates (17) are arranged and that the reactor (01) for solid materials has a valve (P1) for the removal of sediment.

10. Device according to claim 9, characterized by two inclined worm presses (161, 162) operating in opposite directions and/or a table or table plates designed as heat exchangers (02).

11. Device according to one of claims 9 or 10, characterised in that the reactor (01) for solid materials is equipped with at least one screen (26), preferably several slotted hole screens with different mesh sizes arranged one on top of each other, arranged before the outlet and/or has a gas evacuation system which is preferably connected with a compost filter (15).

12. Device according to one of claims 9 to 11, characterized in that the worm press (16; 161, 162) arranged in the upper third of the reactor bottom is equipped with an exchangeable slotted hole screen (26), whose slotted openings run parallely to the conveying direction and/or that the slotted hole screen is at a decreasing distance from the periphery of the rotating worm shaft (16; 161, 162), whereby preferably the distance between the slotted hole screen (26) and the conveyor worm (16; 161, 162) and the contact pressure can be modified due to a changeable suspension of the worm press.

13. Device according to claim 12, characterized in that the water squeezed out by the worm press (16; 161, 162) is collected and drained off into a collecting basin (25) with an inclined bottom, having a rinsing device and/or that the conveying direction changes by changing the worm windings above the pressing zone, thereby increasing the compression effect, whereby preferably at the deepest point of the reactor bottom there is also an opening which can be closed at will, for the extraction of the squeezed out water.

14. Device according to one of claims 9 to 13, characterized by flexible elements such as brushes, rubber bands etc. at the margin of the worm shafts (16; 161, 162) for cleaning and prevention of clogging of the openings of the slotted hole screen and the discharge openings and/or by a changeable positioning angle of the reactor (01) for solid materials, particularly the bottom inclination.

15. Device according to one of claims 9 to 14, characterized in that the reactor (01) for solid materials has a preferably closable filling funnel (19) and a troughed chain conveyor (27) transporting the bio-residuals, which are advantageously built gastight and/or with an active air exhaust by means of side channel fans, whereby preferably between the reactor (01) for solid materials and the anaerobic reactor (08) a sedimentation tank (05) is arranged, which preferably has an outlet through which the resulting sludge can be discharged and directly sent back to the reactor (01) for solid materials and/or whereby the reactor (01) for solid materials and/or the sedimentation tank (05) are provided with an aeration circuit or an air supply system (03).

16. Device according to one of claims 9 to 15, characterized in that between the sedimentation tank (05) and the anaerobic reactor (08) a degassing reactor, preferably an ultrasound reactor (06) or a biostripper is arranged and/or that the anaerobic reactor (08) is designed as a solid-bed reactor and/or has several grid supports promoting surface growth, said grids preferably having a rough surface.

17. Device according to one of claims 9 to 16, characterized in that the anaerobic reactor (08) has a gas dome, preferably with a gas separator and/or that several solid-bed reactors (08a, b, c) are provided, which are interconnected by communicating pipes.

## Revendications

1. Procédé de traitement de matières résiduelles biologiques organiques (31, 33 à 35), en particulier de déchets communaux et'ou industriels (33) y compris des restes crues et'ou cuites de repas (34), des déchets agricoles, en particulier excréments animaux et/ou composants végétaux, dans le cas duquel les matières résiduelles biologiques (31, 33 à 35) sont séparées en une fraction solide et une fraction liquide, et la fraction solide est amenée par la suite à un compostage aérobie de meule et la fraction liquide est amenée à un réacteur anaérobie duquel est extrait le biogaz généré par fermentation,
**caractérisé par le fait que**
les composants contenant C (organiques) dissolus et solubles dans l'eau (DOC) sont enlevés par lavage des matières résiduelles biologiques (31, 33 à 35) avant la séparation solide-liquide, tout en mélangeant et homogénéisant de manière intensive.

2. Procédé selon la revendication 1, caractérisé par le fait que, dans l'étage de séparation a), l'humidité initiale ou l'humidité de 70 à 80 pourcent volumétrique, mais de 40 à 60 pourcent volumétrique du moins, est maintenue durant l'enlèvement par lavage de composants organiques dissolus dans l'eau, le cas échéant par l'amenée d'eau (44), de préférence, l'eau (44) libérée de composants contenant C au moyen de la méthanisation effectuée étant menée de façon recyclée du réacteur anaérobie (08) à l'étage de séparation a).

3. Procédé selon l'une des revendications 1 ou 2, caractérisé par le fait que, dans l'étage de séparation a), de l'oxygène (011) est amené de manière contrôllée et/ou qu'une température allant jusqu'à 70°C est maintenue constamment, le cas échéant, avec la mesure subséquente d'amener finalement de la chaleur pour générer une température comprise entre 80°C et 90°C, afin d'effectuer une hygiénisation aussi complète que possible, et/ou que la chaleur générée durant les réactions exothermiques, dans l'étage de séparation est evacuée et conduite dans le réacteur anaérobie (08).

4. Procédé selon l'une des revendications 1 à 3, caractérisé par le fait que ce n'est qu'après la teneur en composants dissolus contenant C dans le liquide (41) est en dessous d'un seuil prédéterminable que les matières résiduelles restantes sont pressurées de manière à atteindre une teneur restante en humidité aussi faible que possible, avant que l'ensemble de la charge de matières résiduelles soit déchargé de l'étage de séparation et par la suite composté, la séparation de composants liquides (41) et de matières résiduelles (37, 39) étant effectuée de préférence par pressurage et/ou par tamisage.

5. Procédé selon l'une des revendications 1 à 4, caractérisé par le fait que le liquide (41) qui contient des composants contenant C et qui est présent en tant que suspension, est soumis à la sédimentation avant d'être amené au réacteur anaérobie (08), de préférence, la matière solide résultant en tant que boue durant la sédimentation étant amenée comme agent d'inoculation pour accélérer la réaction, de préférence après l'enrichissement en oxygène après un remplissage frais de matières résiduelles biologiques.

6. Procédé selon l'une des revendications 1 à 5, caractérisé par le fait que le liquide (41) évacué de l'étage de séparation est - avant l'introduction dans le réacteur anaérobie (08) - soumis à un traitement par ultrasons ou à un stripage de biogaz afin de supprimer l'oxygène encore contenu, et/ou que des particules solides encore contenues dans le liquide (suspension) sont extraites de temps en temps en tant que boues de curage du réacteur anaérobie (08), le cas échéant, avec des quantités faibles en métal, en particulier en métal lourd, d'abord dissolues et plus tard combinées contenues dans des précipitations.

7. Procédé selon l'une des revendications 1 à 6, caractérisé par le fait que l'on maintient, dans le réacteur anaérobie (08), durant le traitement avec des bactéries mésophiles une température qui est comprise entre 45 et 40°C, tandis que dans le cas d'un traitement avec des bactéries thermophiles la température maintenue est comprise entre 50 et 55°C.

8. Procédé selon l'une des revendications 1 à 7, caractérisé par le fait que le réacteur anaérobie (08) est rempli de manière continue ou à un intervalle de 1 à 2 heures du liquide (41) évacué de l'étage de séparation.

9. Dispositif destiné à la production de biogaz (42) et de compost (37, 40) par fermentation par voie humide et par compostage de matières résiduelles biologiques organiques (31, 33 à 35) comprenant un réacteur (01) pour matières solides qui est réalisé en tant que séparateur solide-liquide à vis de pressage et qui possède une sortie de liquide et un réacteur anaérobie (08) monté à la suite, destiné à la fermentation et présentant un évent de biogaz, ainsi qu'un dispositif de compostage, caractérisé en ce que le réacteur (01) pour matières solides présente une presse en vis (16) disposée de manière inclinée, en ce qu'une table ou des plaques de table (17) sont disposées au-dessus de cette presse en vis (16) et parallèlement à celle-ci, et en ce que le réacteur (01) pour matières solides présente une soupape (P1) destiné à la prise de sédiment.

10. Dispositif selon la revendication 9, caractérisé par deux presses en vis (161, 162) inclinées et opposées et/ou une table ou des plaques de table (17) réalisées en tant qu'échangeurs de chaleur (02).

11. Dispositif selon l'une des revendications 9 ou 10, caractérisé par le fait que le réacteur (01) pour matières solides est équipé d'un tamis (26) du moins, de préférence de plusieurs tamis à fentes présentant un nombre mesh différent et disposés l'un au-dessus de l'autre, qui sont arrangés devant la sortie de liquide, et/ou qu'il dispose d'un dispositif d'évacuation de gaz qui est, de préférence, relié à un filtre de compost (15).

12. Dispositif selon l'une des revendications 9 à 11, caractérisé par le fait que la presse en vis (16; 161, 162) disposée dans le tiers supérieur du fond du réacteur est pourvue d'un tamis à fentes (26) échangeable dont les ouvertures fendues s'étendent parallélement à la direction de transport, et/ou que le tamis à fentes présente une distance se rétrécissant par rapport à la circonférence de l'arbre en vis tournant (16; 161, 162), de préférence, la distance entre le tamis à fentes (26) et l'hélice transporteuse (16; 161, 162) ainsi que la pression de serrage pouvant être modifiée par une suspension réglable de la presse en vis.

13. Dispositif selon la revendication 12, caractérisé par le fait que l'eau libérée par la presse en vis (16; 161, 162) est recueillie et dérivée dans une cuve de réception (25) pourvue d'un dispositif de rinçage et présentant un fond incliné, et/ou que le dispositif de transport change par changement de la spire de la vis au-dessus de la zone de pressage ce qui a une augmentation de l'effet de pressage pour conséquence, de préférence, au point le plus bas du fond du réacteur étant disposée également une ouverture qui peut être fermée à souhait et qui est destinée à la prise d'eau extraite par pressage.

14. Dispositif selon l'une des revendications 9 à 13, caractérisé par des dispositifs flexibles tels que brosses, rubans en caoutchouc etc. disposés sur la périphérie des arbres en vis (16; 161, 162) et destinés au nettoyage et à prévenir des obstructions des ouvertures du tamis à fentes et de celles de décharge et/ou en ce que l'angle de positionnement du réacteur (01) pour matières solides, en particulier l'inclinaison du fond est changeable.

15. Dispositif selon l'une des revendications 9 à 14, caractérisé par le fait que le réacteur (01) pour matières solides présente un entonnoir de remplissage (19) qui, de préférence, peut être fermé ainsi qu'un convoyeur à auges à chaîne (27) transportant les matières résiduelles biologiques, qui est réalisé de préférence de manière à être étanche aux gaz et/ou avec une aspiration d'air active au moyen de compresseurs de canal latéral, de préférence, entre le réacteur (01) pour matières solides et le réacteur anaérobie (08) étant monté un réservoir de sédimentation (05) qui, de préférence, est pourvu d'une sortie au travers de laquelle les boues en résultant peuvent être déchargées et amenées à nouveau directement au réacteur (01) pour matières solides, et/ou le réacteur (01) pour matières solides et/ou le réservoir de sédimentation (05) étant pourvus d'un circuit d'aération ou de dispositifs d'amenée d'air (03).

16. Dispositif selon l'une des revendications 9 à 15, caractérisé par le fait qu'entre le réservoir de sédimentation (05) et le réacteur anaérobie (08) est disposé un réacteur de dégazage, de préférence un réacteur à ultrasons (06) ou un stripper de biogaz, et/ou que le réacteur anaérobie (08) est réalisé en tant que réacteur de lit solide et/ou présente plusieurs supports de grille favorisant l'accroissance, qui possèdent de préférence une surface rugueuse.

17. Dispositif selon l'une des revendications 9 à 16, caractérisé par le fait que le réacteur anaérobie (08) présente un dôme de gaz, de préférence avec un séparateur de gaz, et/ou que l'on prévoit plusieurs réacteurs de lit solide (08a, b, c) qui communiquent entre eux par l'intermédiaire de tubes communicantes.
